# EUROPÄISCHE PATENTSCHRIFT

(11) **EP 0 668 768 B1**
(45) Veröffentlichungstag und Bekanntmachung des Hinweises auf die Patenterteilung: **27.08.1997**
(21) Anmeldenummer: 93924503.1
(22) Anmeldetag: 05.11.1993
(51) Int. Cl.: A61K 35/78

(54) **ZUSAMMENSETZUNG ZUR BEKÄMPFUNG VON DERMATOMYKOSEN UND DEREN ERREGERN SOWIE VON SCHWEISSBILDUNG UND KÖRPERGERUCH**
COMPOSITION FOR CONTROLLING DERMATOMYCOSES AND THEIR AGENTS, AS WELL AS TRANSPIRATION AND BODILY ODOURS
COMPOSITION PERMETTANT DE LUTTER CONTRE LES DERMATOMYCOSES ET LEURS AGENTS CAUSALS, AINSI QUE CONTRE LA SUDATION ET LES ODEURS CORPORELLES

(30) Priorität: 06.11.1992 DE 4237551; 12.02.1993 DE 4304284
(43) Veröffentlichungstag der Anmeldung: 30.08.1995
(73) Patentinhaber: KÖNIGER, Helmut, D-80636 München (DE)
(72) Erfinder: KÖNIGER, Helmut, D-80636 München (DE)
(74) Vertreter: Turi, Michael, Dipl.-Phys.
(86) Internationale Anmeldenummer: DE9301061
(87) Internationale Veröffentlichungsnummer: WO9411009

(56) Entgegenhaltungen:
- EP-A- 0 248 701
- FR-A- 2 567 025
- FR-A- 2 610 523
- FR-A- 2 662 078
- 'ROTE LISTE' 1965 , EDITO CANTOR , AULENDORF/ WüRTT DE siehe Seite 1074 " Solum uliginos. comp. "Wala""

## Beschreibung

Die Erfindung betrifft eine Zusammensetzung, die Bestandteile des Krauts der Gattung Equisetum und Bestandteile des Krauts und/oder der Blüte der Gattung Lavandula enthält, deren Verwendung zur äußerlichen Vorbeugung gegen und Bekämpfung von Dermatomykosen, insbesondere Dermatophytosen, von Hauterkrankungen mit dem klinischen Erscheinungsbild von Tinea pedis und von deren Erregern hervorgerufenen Hauterkrankungen an anderen Körperstellen, sowie von Bromhidrosis, sonstiger Schweißbildung und unangenehmem Körpergeruch; die Herstellung einer Zusammensetzung zur Bekämpfung von Erregern von Dermatomykosen und Erregern, die das klinische Erscheinungsbild von Tinea pedis hervorrufen können, in Textilien, sonstigen Bekleidungsstücken und Hygieneartikeln, die mit der Haut in Berührung stehen, sowie zur Deodorierung derselben, Verfahren zur Erreichung der genannten Wirkungen sowie ein Verfahren zur Herstellung der erfindungsgemäßen Zusammensetzung.

Dermatomykosen und speziell Dermatophytosen sind Krankheiten, die nicht nur weit verbreitet sind, sondern wegen ihrer häufigen kosmetischen Implikationen den Patienten auch psychisch sehr beeinträchtigen können. Insbesondere Tinea pedis ("Fußpilz") ist eine als Volkskrankheit zu bezeichnende Dermatomykose, die als sehr unangenehm empfunden wird. Ihre Häufigkeit wird in Europa und Nordamerika auf 15-30% der Bevölkerung geschätzt. Verursacht wird sie hauptsächlich von allgegenwärtigen Dermatophyten, am häufigsten von Trichophyton rubrum, Trichophyton mentagrophytes und Epidermophyton floccosum. Sie kann sich auf Nägel (Tinea unguium), Hände (Tinea manus) und andere Körperteile ausdehnen und mit bakteriellen Infektionen und/oder einer Pilzinfektion durch Candida einhergehen, oder ein Hauterkrankung mit einem von Tinea pedis praktisch nicht zu unterscheidenden klinischen Erscheinungsbild, die ebenfalls auf andere Körperstellen ausgedehnt sein kann, kann gänzlich von den einer oder beiden der letzeren Infektionen verursacht sein. Die Differentialdiagnose zum Nachweis von Dermatophyten ist schwierig (die Kultur von Dermatophyten erfordert 2 - 4 Wochen bei Raumtemperatur) und wird meistens nicht gestellt. Sehr häufig ist die Krankheit von Bromhidrosis (übelriechendem Schweiß), die nach allgemeiner Ansicht von Bakterien verursacht wird, begleitet und bildet so auch ein ernsthaftes kosmetisches Problem.

Prädisponierende Faktoren, insbesondere für Tinea pedis, sind Hyperhidrosis (übermäßige Schweißabsonderung) und Akrozyanose, die häufig mit örtlicher Hyperhidrosis einhergeht.

Die Krankheit verläuft ohne Behandlung meist chronisch. Aber auch mit den derzeit zur Verfügung stehenden (meist langwierigen) Behandlungsmethoden und unter Beachtung aller notwendigen Hygienevorschriften ist sie manchmal kaum ausheilbar, und Rezidive sind, insbesondere wenn die oben erwähnten prädisponierenden Faktoren vorhanden sind, sehr häufig (zu einer Übersicht der derzeitigen Behandlungsmethoden von Dermatomykosen siehe O. Braun-Falco et al., Dermatology, Springer Verlag, Berlin, 1991, Seiten 219 bis 246, insbesondere Differentialdiagnose von Tinea pedis Seite 228 und Behandlungsmethoden dafür Seiten 230 bis 232).

Auch die erforderliche Desinfektion der Bekleidung, insbesondere der Schuhe, ist nicht unproblematisch. Meist werden Formaldehyd-haltige Lösungen empfohlen, die jedoch Allergien auslösen können und auch wegen der bekannten Gesundheitsrisiken von Formaldehyd nicht als Ideallösung betrachtet werden können.

Unangenehmer Körpergeruch entsteht sowohl durch Absonderung übelriechender Stoffe vom Körper als auch durch die Einwirkung von Bakterien und/oder Sauerstoff auf vom Körper ausgeschiedene, ursprünglich wenig bis nicht riechende Stoffe, die dabei in stark riechende Zersetzungprodukte überführt werden.

Die Geruchsbildung kann einmal dadurch bekämpft werden, daß die Absonderung von Ausscheidungsprodukten durch den Körper, insbesondere im Schweiß, verringert bis unterbunden wird. Dies führt naturgemäß zu einem deodorierenden Effekt. Weiter kann die Geruchsbildung auch noch bekämpft werden, indem bereits ausgeschiedene Absonderungsprodukte daran gehindert werden, einen üblen Geruch zu entwickeln, was hauptsächlich durch a) Abtöten der zersetzenden Bakterien, b) Verhinderung der erfolgreichen Einwirkung von Sauerstoff (Oxidation) unter Umwandlung in olfaktorische Komponenten und c) chemisches oder physikalisches Bindung bereits gebildeter olfaktorischen Komponenten erzielt werden kann.

Es sind zahlreiche kosmetische Mittel zur Bekämpfung von Körpergeruch auf dem Markt, die jedoch alle gewisse Nachteile aufweisen. Die Mittel, die vor allem die Schweißabsonderung hemmen, wie Adstringentien auf der Grundlage von Aluminiumsalzen und insbesondere auf der Grundlage von Aluminiumhydroxychlorid, entfalten meist ihre Wirkung nur bei längerem Gebrauch, also nicht sofort in einer Situation, bei der eine Schweißreduzierung erwünscht ist, beispielweise in einer Situation starker körperlicher oder nervlicher Belastung; außerdem führt der notwendige Dauergebrauch dazu, daß sich die Poren ständig in einem verengten Zusatnd befinden, auch wenn eine Schweißhemmung gar nicht erforderlich ist. Dagegen wirken kosmetische Mittel mit deodorierender Wirkung in engerem Sinn wenig oder gar nicht auf das Schweißvolumen ein, sondern verhindern eine Geruchsbildung durch bereits ausgeschiedene Körperabsonderungen hauptsächlich dadurch, daß sie zur Zerstörung der schweißzersetzenden Bakterien führen, also bakterizid oder aseptisch wirken.

Wünschenswert wäre jedoch ein Mittel, das sowohl im Bedarfsfall das Schweißvolumen verringert, ohne dies auf Dauer zu tun, als auch die unangenehme Geruchsbildung durch bereits ausgeschiedene Körperabsonderungen verhindert.

Aufgabe der Erfindung ist es demnach, ein Mittel zu schaffen, das Dermatomykosen, insbesondere Dermatophytosen, und Hauterkrankungen mit einem Tinea pedis gleichenden klinischen Erscheinungsbild, die auch an anderen Körperstellen auftreten können, damit verbundene Bromhidrosis und den prädisponierenden Faktor Hyperhidrosis wirksam bekämpft und auch zur Desinfektion von Bekleidung wirksam ist sowie allgemein als schweißhemmendes und deodorierendes Mittel brauchbar ist.

Diese Aufgabe wird durch die Zusammensetzung des Anspruchs 1 gelöst.

Weiter betrifft die Erfindung die Verwendung der Zusammensetzung des Anspruchs 1 zur äußerlichen Vorbeugung gegen und Bekämpfung von Dermatomykosen, insbesondere Dermatophytosen, Hauterkrankungen mit dem klinischen Erscheinungsbild von Tinea pedis und von deren Erregern hervorgerufenen Hauterkrankungen an anderen Körperstellen, zur Bekämpfung von Bromhidrosis, Schweißbildung und als deodorierendes Mittel sowie zur Bekämpfung der Erreger der obigen Erkrankungen in Textilien, sonstigen Bekleidungsstücken und Hygieneartikeln, die mit der Haut in Kontakt stehen, und Deodorierung derselben.

Die Erfindung betrifft auch Verfahren zum Erreichen der genannten Wirkungen unter Verwendung der erfindungsgemäßen Zusammensetzung sowie ein Verfahren zur Herstellung der erwähnten Zusammensetzung.

Auf der Suche nach einem wirsamen und schnell wirkenden Mittel gegen Darmatomykosen, insbesondere gegen die Volkskrankheit mit dem klinischen Erscheinungsbild von Tinea pedis und damit verwandten Hauterkrankungen, sowie gegen Schweißabsonderung und unangenehmen Körpergeruch wurde überraschenderweise gefunden, daß eine Zusammensetzung, die Bestandteile der grünen Teile von Equisetum und Bestandteile des Strauchs und/oder der Blüten von Lavandula enthält, nicht nur innerhalb weniger Tage die Hautinfektion ausheilt, sondern darüber hinaus ausgezeichnet Hyperhidrosis, unangenehmen Körpergeruch und Bromhidrosis bekämpft und meist bereits nach einmaliger Anwendung Bekleidungsstücke, insbesondere auch Schuhe, so wirksam desinfiziert, daß eine Reinfektion nicht stattfindet, und diese ebenfalls hervorragend deodoriert.

Equisetum (Schachtelhalm), insbesondere die Arten Equisetum arvense (im Volksmund auch Zinnkraut genannt, da diese Art, die als Unkraut weit verbreitet ist, früher zum Reinigen von Zinngeräten verwendet wurde) und Equisetum hiemale, wurden vermutlich bereits im Altertum als Heilpflanzen verwendet. In jüngerer Zeit wurde Equisetum vor allem durch Kneipp wieder in Erinnerung gebracht.

Gerhard Madaus berichtet in seinem "Lehrbuch der biologischen Heilmittel", Georg Thieme Verlag, Leipzig, 1938, Seiten 1267 - 1278, über unzählige innere und äußere, im Volksbrauch überlieferte Anwendungen von Equisetum-Arten, von denen im vorliegenden Zusammenhang vor allem die adstringierende, blutstillende Wirkung, die sich wiedersprechen erscheinenden Berichte über eine schweißtreibende Wirkung (offensichtlich bei innerer Anwendung; G. Madaus, a.a.O., Seite 1273) und über eine Bekämpfung von Schweißfüßen mit Fußbädern und Umschlägen sowie die Verwendung zur Bekämpfung von Fluor albus, Ozäna, Rachitis und Gingivitis (G. Madaus, a.a.O., Seite 1273) mit Aufgüssen und Abkochungen von Equisetum interessieren.

Auch ein neueres, volkstümliches Heilkräuterbuch (Maria Treben, "Gesundheit aus der Apotheke Gottes", Eigenverlag "Verein Freunde der Heilkräuter", ohne Jahr (aber nach 1978)) erwähnt ebenfalls die blutstillende Wirkung, die Wirkung gegen Schweißfüße und die Verwendung bei entzündlichen Erkrankungen im Mund- und Halsbereich von Zinnkraut (Maria Treben, a.a.O., Seiten 42 und 43). In Joseph Karl, "Phytotherapie", Verlag Tibor Marczell, München, 4. Auflage 1983, Seite 133, wird die äußerliche Anwendung von Equisetum arvense bei Dermatosen, Pemphigus, Dekubitus, alten Wunden und Ulcus cruris erwähnt, in Hans Braun, Dietrich Frohne "Heilpflanzen-Lexikon für Ärzte und Apotheker", Gustav Fischer Verlag, Stuttgart, 1987, Seite 105, werden Abkochungen bzw. ein Extrakt von Equisetum arvense als äußerliches Mittel gegen Dekubitus und rheumatische Beschwerden aufgeführt.

Die wirksamen Inhaltsstoffe des Krauts von Equisetum arvense sind laut H. Braun, D. Frohne, a.a.O, Kieselsäure (zum Teil in löslicher Form), Flavanoide (Quercetin und Kämpferol als Aglyka), Kaliumsalze und, mit Fragezeichen versehen, Saponine, während J. Karl, a.a.O., und G. Madaus, a.a.O., Seite 1275, das Vorhandensein von Saponinen als gesichert ansehen.

Auch die Verwendung verschiedener Lavandula-Arten als Heilpflanzen läßt sich zumindest bis ins 12. Jahrhundert zurückverfolgen. Dabei steht die Art Lavandula officinalis Chaix et Vill. (= Lavandula angustifolia Mill.; echter Lavendel) im Vordergrund, aber auch andere Arten, insbesondere Lavandula spica (= Lavandula latifolia Vill.), werden bei verschiedenen Autoren erwähnt.

Bei G. Madaus, a.a.O., Seiten 1723 - 1725, finden sich bei der hier interessierenden äußerlichen Anwendung von Lavandula - in Form eines Badezusatzes, des Öls, eines Extrakts, einer Blütenverreibung und/oder einer Tinktur - Hinweise auf antiseptische und eiterwidrige Eigenschaften, auf Wirkungen bei Epilepsie und ohnmachtsanfällen, bei Blutandrang, Rheuma, Gicht, Neuralgien, Ischias und auch bei Skabies und Fluor albus. Weiter sollen Flores Lavandulae bei Migräne und nervöser Aufregung beruhigend wirken. J. Karl, a.a.O, Seite 203, führt Lavendelöl als mildes Sedativum und Neurostimulans, und, bei äußerlicher Anwendung, als Geruchskorrigens mit beruhigendem Einfluß und als Hyperaemikum auf; H. Braun und D. Frohne, a.a.O., Seite 147, erwähnt es bei äußerlicher Anwendung als Hautreizmittel.

Ältere Literatur führt als verwendeten Pflanzenteil neben der Blüte auch das Kraut auf, während in jüngerer Zeit praktisch nur noch die Blüten verwendet wurden. Die hauptsächlichen wirksamen Inhaltsstoffe von Lavendelöl sind Linalylacetat und weitere Terpene sowie Gerbstoffe.

Die meisten der oben aufgeführten Wirkungen bei äußerlicher Anwendung von Bestandteilen von Equisetum bzw. Lavandula sind naturgemäß ziemlich vage; sie beruhen zum größten Teil auf überliefertem Brauchtum aus einer Zeit, wo eine genauere Diagnose der betreffenden Erkrankungen kaum möglich war.

Beim Testen von Equisetum arvense allein in Form eines verdünnten Extraktes, der über mehrere Tage aufgetragen wurde, zeigte sich, daß die Bildung von Fußschweiß und Schweiß an anderen Körperstellen recht wirkungsvoll unterbunden wurde, wobei allerdings ein starkes, unangenehmes Austrocknen der Haut zu beobachten war, die Hauterkrankung mit dem klinischen Erscheinungsbild von Tinea pedis aber nicht beseitigt wurde.

Auch Lavendel allein, mehrere Tage in Form von reinem oder mit wäßrigem Isopropanol verdünntem Lavandulae aetheroleum aufgetragen, ergab keine Besserung des klinischen Erscheinungsbildes von Tinea pedis; lediglich eine vorübergehende, geruchskorrigierende und allgemein. hautfreundliche Wirkung war zu beobachten.

Erst die über einige Tage aufgetragene Mischung dieser beiden Komponenten zeitigte die überraschende synergistische Wirkung bei der Bekämpfung dieser Erkrankung sowie bei der ohne mit unangenehmen Begeliterscheinungen (Austrocknen der Haut) verbundenen Beseitigung von Schweißbildung und unangenehmem Körpergeruch. Bromhidrosis war nach wenigen Tagen beseitigt und trat nicht wieder auf, und die Hautläsionen heilten vollständig ab. Hyperhidrosis wurde im Behandlungszeitraum so gut wie vollständig unterbunden und blieb auch anschließend signifikant verringert. Rezidive traten im Beobachtungszeitraum (6 Monate) entweder überhaupt nicht auf oder konnten durch erneutes Aufbringen der Mischung im Keime erstickt werden. Eine wirksame Desinfektion der Schuhe, erkennbar am vollständigen Verschwinden üblen Geruches und dem Nicht-Auftreten von Rezidiven, konnte meist bereits nach einmaliger Behandlung derselben mit der Mischung und Einwirkenlassen über 24 Stunden erreicht werden.

Bei diesen Untersuchungen wurde auch festgestellt, daß sich die obige Mischung allgemein hervorragend zur Hemmung von Hyperhidrosis und zur Geruchshemmung eignet. Zwar ist hier auch Equisetum alleine recht wirksam, zeigt aber die unangenehme Nebenwirkung, die Haut zu stark auszutrocknen und spröde zu machen, während Lavandula allein auf der Haut zwar angenehm ist, aber keine bemerkenswerte schweißhemmende Wirkung aufweist. Die Mischung beider Komponenten sorgt für eine rasch eintretende, starke, jedoch nicht zu unangenehmer Austrocknung der Haut führende Unterbindung der Ausdünstung und des unangenehmen Körpergeruchs, wobei die Haut geschmeidig bleibt und - vermutlich aufgrund der olefaktorischen Komponente von Lavendel - eine von den Testpersonen als sehr angenehm und beruhigend beschriebene Wirkung aufweist, was insbesondere in Streßsituationen, in denen häufig eine vegetativ bedingte Hyperhidrosis auftritt, von Vorteil ist. Die Anwendung der Mischung zur Schweißhemmung ist insbesondere auch dort vorteilhaft, wo die handelsüblichen schweißhemmenden Mittel aus praktischen Gründen kaum angewendet werden (zum Beispiel auf den Händen) oder wenn ein praktisch sofortiger Wirkungeintritt erforderlich ist, was bei den am stärksten wirkenden handelsüblichen schweißhemmenden Mitteln, in denen Aluminium-oder andere anorganische Salze mit porenverengender Wirkung die wirksamen Bestandteile sind, nicht der Fall ist.

Der biologische Wirkmechanismus des überraschenden, oben beschriebenen Synergismus ist noch nicht aufgeklärt.

Die Arten Equisetum arvense, Equisetum hiemale und Lavandula officinalis Chaix et Vill. (= Lavandula angustifiola Mill.), Lavandula spica sowie Lavandula hybrida Rev. sind als Ausgangspflanzen für die erfindungsgemäße Zusammensetzung besonders bevorzugt.

Als Ausgangsmaterialien für eine galenische Zubereitung der Mischung aus Equisetum- und Lavadulabestandteilen werden bevorzugt ein ethanolisch-wäßriger (30:70 Vol/Vol) Extrakt (1:1 Vol/Vol) des ersteren und das reine, z.B. durch Wasserdampfdestillation gewonnene Blütenöl der letzeren verwendet. Es sind jedoch auch andere Ausgangsmaterialien möglich, z.B. das getrocknete, zerriebene Kraut, ein aus dem Kraut ausgepreßter Saft oder ein wäßriger Aufguß von Equisetum oder Lavandula oder die zerriebenen Blüten oder ein Extrakt aus den Blüten der letzteren. Eine hervorragende Übersicht über mögliche Aufbereitungsformen, die in diesem Zusammenhang verwendet werden können, findet sich in Paul Heinz List und Peter C. Schmidt, "Technologie pflanzlicher Arzneizubereitung", Wissenschaftliche Verlagsgesellschaft mbH, Stuttgart, 1984.

Die Ausgangsmaterialien können, falls erforderlich, weiter in alle für die äußerliche Anwendung geeignete galenische Formen und alle Formen für die kosmetische und desinfizierende Anwendung gebracht werden, wie z.B. Puder, Salben, Cremes, Gele, Lotionen, Emulsionen, Lösungen, beipielsweise als Sprays auftragbar, und Sprays mit Treibmittel (Aerosole). Eine Übersicht über mögliche Galeniken ist in Karl Thoma, "Dermatika", 2. Auflage, München, 1983 (zu beziehen über Werbe- und Vertriebsgesellschaft Deutscher Apotheker m.b.H., Frankfurt) sowie in Remington's Pharmaceutical Sciences, 18. Auflage, Mack Publishing Company, Easton, Pennsylvania, 1990, insbesondere auch Seiten 1694 - 1712 (Aerosole) zu finden.

Als galenische und für die weiteren Anwendungen geeignete Form der erfingungsgemäßen Zusammensetzung wird eine Lösung eines wie oben zusammengesetzten ethanolisch-wäßrigen Extrakts von Equisetum und Blütenöl von Lavandula in einer Mischung von Ethanol oder Isopropanol und Wasser, insbesondere 35% (Vol/Vol) Isopropanol/Wasser, die bequem mit Hilfe eines Zerstäubers aufgetragen werden kann, besonders bevorzugt.

Das Verhältnis von Equisitumbestandteilen zu Lavandulabestandteilen in der erfindungsgemäßen Zusammensetzung liegt vorzugsweise im Bereich von 15 : 100 bis 100 : 15 Gewichtsteilen. Die Konzentrationen der Pflanzenbestandteile in den erfindungsgemäßen Arznei- und Kosmetikzubereitungen richten sich in erster Linie nach dem Zweck der Anwendung (z.B. Prophylaxe oder Therapie), der Art und Schwere der Erkrankung bzw. kosmetischen Beeinträchtigung sowie nach der galenischen Form. Für Desinfektionszwecke sind Konzentrationen von jeweils ungefähr 1 bis 40% (Vol/Vol) im oben angegebenen Mischungsbereich in einem Trägermittel vorteilhaft. Im kosmetischen Bereich zur Verhinderung von Schweißbildung und unangenehmem Körpergeruch genügen häufig geringere Konzentrationen als die oben angegebenen.

In 35%iger (Vol/Vol) wäßriger Isopropanollösung haben sich ungefähr 1,25 - 10 Vol% Schachtelhalmextrakt (1:1 (Vol/Vol) in 30%-igem (Vol/Vol) wäßrigem Ethanol) und etwa genauso viel unverdünntes Lavendöl als eine in den meisten Fällen vorteilhafte Konzentration für die Therapie von Hauterkrankungen mit dem klinischen Erscheinungsbild von Tinea pedis erwiesen. Eine derartige Lösung wird nach gründlichem Waschen und Abtrocknen der Fü Behandlungsdauer wirkungsvoll bekämpft und zeigt sich auch nach deren Abschluß deutlich verringert.

Zur Desinfektion und Deodorierung der Schuhe wird beispielweise eine Lösung der wie oben beschriebenen Konzentration in dieselben gesprüht, bis sie vollständig damit befeuchtet sind. Nach 24stündigem Einwirken ist im allgemeinen bereits nach dem ersten Einsprühen jeder unangenehme Geruch verschwunden, und die Schuhe können ohne Gefahr einer Reinfektion weiter getragen werden.

Bei der Verwendung der erfindungsgemäßen Zusammensetzung als schweißhemmendes und geruchshemmendes Mittel in der wie oben beschriebenen Isopropanollösung genügt häufig auch eine kleinere Konzentration als die oben angegebene, beispielweise die halbe Konzentration. Bei starker Schweißbildung hat sich ein Überschuß an Equisetum als vorteilhaft erwiesen. Die Lösung wird auf die gewünschte Körperstelle aufgesprüht, bis diese mit einem Flüssigkeitsfilm überzogen ist, dann läßt man sie eintrocknen. Die Schweißbildung wird selbst bei größter körperlicher Anstrengung über mindestens 30 Minuten so gut wie vollständig unterbunden. Im allgemeinen halten Schweiß- und Geruchshemmung über mehrere Stunden an.

Die erfindungsgemäße Zusammensetzung kann neben Trägerstoffen und anderen pharmazeutischen Hilfsstoffen auch noch weitere Beistoffe (einschließlich weiterer medizinischer, kosmetischer und keimhemmender bzw. -tötender Mittel) enthalten. Z.B. kann Phthalsäure zugesetzt werden, was ein längeres Anhalten des Duftes von Lavendelöl bewirkt. Auch andere Pflanzenbestandteile, wie z.B. Fichtennadelöl als weitere Geruchs- und Gerbkomponente, oder Parfumstoffe können beigemischt sein.

Im folgenden wird die Erfindung anhand von Beispielen weiter erläutert, die jedoch die Erfindung nicht beschränken.

### Beispiel 1

Bestandteile des Krauts von Equisetum arvense wurden in Form eines Extrakts eingesetzt (Schachtelhalmkraut-Extrakt, Fluidextrakt 1:1 (Vol/Vol), Auszugsmittel 30%-iges( Vol/Vol) wäßriges Ethanol, erhältlich von Dr. Hetterich KG, Fürth).

Bestandteile von Lavandula officinalis wurden in Form von durch Wasserdampfdestillation gewonnenem Blütenöl mit einem Linanylacetatgehalt von 42,1 Gew.-% (DAB) [Deutsches Arzneibuch] eingesetzt (Lavendöl (Lavandulae aetheroleum) DAB Mont Blanc 38-45%, erhältlich von Vaselin-Werke Wasserfuhr GmbH, Bonn).

Die obigen Bestandteile wurden in 35%-igem (Vol/Vol) wäßrigem Isopropanol unter Schütteln in einer Zerstäuberflasche gelöst. Die Lösung war in einigen Fällen leicht getrübt, was darauf hinwies, daß ein Teil der Ausgangsmaterialien als Emulsion vorlag. Die Emulsion entmischte sich jedoch auch bei längerer Aufbewahrung nicht.

Zubereitungen der folgenden Konzentrationen der obigen Ausgangsmaterialien in 35%-igem (Vol/Vol) wäßrigem Isopropanol wurden hergestellt:

| Zubereitungs-Nr. | Schachtelhalm-Extrakt (1:1) | Lavendelöl |
|---|---|---|
| 1 | 10% (Vol/Vol) | 10% (Vol/Vol) |
| 2 | 3% (Vol/Vol) | 3% (Vol/Vol) |
| 3 | 7% (Vol/Vol) | 3% (Vol/Vol) |
| 4 (Vergleich) | 20% (Vol/Vol) | - |
| 5 (Vergleich) | - | 20% (Vol/Vol) |
| 6 (Vergleich) | 7% (Vol/Vol) | - |
| 7 (Vergleich) | - | 7% (Vol/Vol) |

Den Proben 1, 2, 3, 5 und 7 wurde zusätzlich jeweils 0,8% (Gew/Vol) Phthalsäure zugesetzt, um die olefaktorische Wirkung von Lavendöl zu verlängern.

### Beispiel 2

Vier Probanden mit einem ausgeprägten klinischen Erscheinungsbild von Tinea pedis, die sich bereits über die Zehenzwischenräume hinaus ausgebreitet hatte und ohne Erfolg mit im Handel befindlichen Medikamenten behandelt worden war (eine Differentialdiagnose war in keinem der Fälle gestellt worden), wurde morgens, mittags und abends nach gründlichem Waschen und Abtrocknen der Füße die erfindungsgemäße Zubereitung Nr. 1 von Beispiel 1 auf die befallenen Stellen des Fußes gesprüht, bis diese von einem Film der Zubereitung überzogen waren. Nach vollständiger Verflüchtigung der Lösungsmittel wurden Strümpfe und Schuhe angelegt.

Bromhidrosis und Juckreiz/Brennen waren nach 3, 4 (2 Probanden) bzw. 7 Tagen vollständig verschwunden. Die Hautläsionen heilten vollständig ab. Während des Behandlungszeitraums war die Bildung von Fußschweiß praktisch vollständig unterbunden und auch nach Beendigung der Behandlung berichteten alle Probanden von einer beachtlichen Verringerung der Schweißbildung. Im Beobachtungszeitraum (6 Monate) trat ein Rezidiv auf (vermutlich durch Infektion in einem Schwimmbad), das jedoch durch eine Wiederholung der obigen Behandlung über 3 Tage im Keime erstickt wurde.

### Beispiel 3

Sämtliche Schuhe der Probanden des Beipiels 2 wurden bis zur guten Befeuchtung mit der erfindungsgemäßen Zubereitung Nr. 1 von Beispiel 1 eingesprüht und 24 Stunden stehengelassen.

Lediglich in einem Fall war der typische Tinea-pedis-Geruch nicht ganz beseitigt, und die Behandlung wurde wiederholt. Die Schuhe wurden nach Ausheilen der Tinea pedis wieder getragen, und bis auf den einen oben erwähnten Fall, der aber vermutlich nicht auf die Schuhe zurückzuführen war, fand keine Reinfektion statt.

### Vergleichsbeispiel 1

Zwei der Probanden des Beispiels 2 wurden vor der Behandlung mit der erfindungsgemäßen Zubereitung Nr. 1 von Beispiel 1 mit der Zubereitung Nr. 4 (Vergleichszubereitung) 7 Tage auf gleiche Weise wie in Beipiel 2 behandelt. Die Schweißbildung wurde auf ähnliche Weise wie in Beispiel 2 unterbunden, allerdings von einem ausgeprägten Austrockenungsgefühl begleitet, der Geruch nach Tinea pedis sowie Juckreiz und Brennen verschwanden jedoch nicht vollständig. Nach Beendigung der Behandlung kehrten die Symptome von Tinea pedis vollständig zurück.

### Vergleichsbeispiel 2

Zwei weitere Probanden des Beispiels 2 wurden vor der Behandlung mit der erfindungsgemäßen Zubereitung Nr. 1 von Beispiel 1 mit der Zubereitung Nr. 5 (Vergleichszubereitung) 7 Tage auf gleiche Weise wie in Beipiel 2 behandelt. Eine signifikante Schweißverminderung trat nicht ein, Juckreiz und Brennen waren, wenn auch in reduziertem Umfang, noch verhanden, lediglich der Geruch von Tinea pedis wurde durch den Lavendel-Geruch übertönt. Nach Beendigung der Behandlung kehrten die Symptome von Tinea pedis vollständig zurück.

### Beispiel 4

A. Zwei Tennisspielern, die während des Spielens durch extreme Schweißbildung an den Händen behindert wurden, wurde die erfindungsgemäße Zubereitung Nr. 3 von Beipiel 1 nach ungefähr 30minütigem Spiel auf die Hände gesprüht, bis sich ein Film der Zubereitung ausgebildet hatte. Man ließ den Film einige Minuten trocknen. Nach ungefähr 4 bis 6 Minuten war keine weitere Schweißbildung mehr zu beaobachten. Die Hände der beiden Spieler blieben über einen Zeitraum von mindestens 30 Minuten so gut wie trocken.
   Dieses Vorgehen wurde mit den Vergleichszubereitungen Nr. 6 und 7 wiederholt. Die Vergleichszubereitung Nr. 6 verhinderte zwar ebenfalls einen Schweißbildung über den oben genannten Zeitraum, aber beide Spieler bemängelten ein zu starkes Trockenheitsgefühl in den Händen, das sich beim Halten des Schlägers unangenehm bemerkbar machte. Die Vergleichslösung Nr. 7 wurde zwar als angenehm auf der Haut empfunden, verhinderte eine Schweißbildung aber nicht signifikant.
B. Drei Probandinnen, die an vegetativ bedingter Hyperhidrosis an den Händen litten, die sich im Beruf sehr störend bemerkbar machte, sprühten sich nach Bedarf entweder die erfindungsgemäße Zubereitung Nr. 2 oder die Vergleichszubereitungen Nr. 6 bzw. Nr. 7 ohne Kenntnis der Inhaltsstoffe auf die Hände. Übereinstimmend wurde berichtet, daß die erfindungsgemäße Zubereitung Nr. 2 die beste Kombination aus Schweißhemmung und Geschmeidigkeitsgefühl verschaffte, während die Vergleichszubereitung Nr. 6 als zu austrocknend und die Vergleichszubereitung Nr. 7 als zu wenig schweißhemmend beschrieben wurden.

### Beispiel 5

Drei männliche Probanden wuschen sich 4 Tage lang morgens mit gewöhlicher, unparfümierter Seife die Achselhöhlen und sprühten anschließend die Zubereitung Nr. 2 des Beispiels 1 in die linke Achselhöhle, während die rechte Achselhöhle unbehandelt blieb. Alle drei Probanden verichteten anschließend ihre übliche (körperlich relativ anstrengende) Arbeit. Nach 6 Stunden wurde der Geruch der jeweils linken und rechten Achselhöhlen verglichen. Dabei zeigte sich, daß die erfindungsgemäß behandelten linken Achselhöhlen deutlich weniger nach Schweiß rochen als die unbehandelten rechten Achselhöhlen. Das Deodorant wurde übereinstimmend als sehr hautfreundlich beurteilt.

### Beispiel 6

Die Schuhe von 2 Personen, die einen Fußschweißgeruch aufwiesen, wurden bis zur guten Befeuchtung mit der Zubereitung Nr. 3 vom Beispiel 1 eingesprüht und 24 Stunden stehengelassen. Der Geruch nach Fußschweiß war in beiden Fällen vollständig verschwunden.

## Patentansprüche

1. Zusammensetzung, dadurch gekennzeichnet, daß sie Bestandteile des Krauts von Pflanzen der Gattung Equisetum und des Krauts und/oder der Blüte von Pflanzen der Gattung Lavandula enthält.

2. Zusammensetzung nach Anspruch 1, dadurch gekennzeichnet, daS die Bestandteile von Pflanzen der Gattung Equisetum durch Extraktion gewonnen wurden und die Bestandteile der Gattung Lavandula ein Blütenöl sind.

3. Zusammensetzung nach Anspruch 1 oder 2, dadurch gekennzeichnet, daß der Extrakt durch Extraktion mit 30%-igem (Vol/Vol) wäßrigem Alkohol gewonnen wurde.

4. Zusammensetzung nach einem der Ansprüche 1 bis 3, dadurch gekennzeichnet, daS die Pflanzen der Gattung Equisetum Pflanzen der Arten Equisetum arvense und/oder Equisetum hiemale und die Pflanzen der Gattung Lavandula Pflanzen der Arten Lavandula officinalis Chaix et Vill. (= Lavandula angustifolia Mill.; echter Lavendel), Lavandula spica (= Lavandula latifolia Vill.) und/oder Lavandula hybrida Rev. sind.

5. Zusammensetzung nach einem der Ansprüche 1 bis 4, dadurch gekennzeichnet, daß die Equisetumbestandteile zu den Lavandulabestandteilen im Bereich von 15 : 100 bis 100 : 15 Teile (Gew./Gew.) vorliegen.

6. Zusammensetzung nach einem der Ansprüche 1 bis 5, dadurch gekennzeichnet, daß sie mindestens ein Trägermittel enthält.

7. Zusammensetzung nach Anspruch 6, dadurch gekennzeichnet, daß das Trägermittel ein zur äußerlichen Anwendung pharmazeutisch und/oder kosmetisch annehmbares Trägermittel ist.

8. Verwendung der Zusammensetzung nach einem der Ansprüche 1 bis 7 bei der Herstellung eines Medikaments zur äußerlichen Vorbeugung gegen und Bekämpfung von Dermatomykosen, insbesondere Dermatophytosen, Hauterkrankungen mit dem klinischen Erscheinungsbild von Tinea pedis und von deren Erregern hervorgerufenen Hauterkrankungen an anderen Körperstellen.

9. Verwendung der Zusammensetzung nach einem der Ansprüche 1 bis 7 bei der Herstellung eines Medikaments oder Kosmetikums zur Bekämpfung von Bromhydrosis, Schweißbildung und bei der Herstellung eines Deodorants.

10. Verwendung der Zusammensetzung nach einem der Ansprüche 1 bis 7 bei der Herstellung eines Mittels zur Bekämpfung von Dermatomykoseerregern und Erregern von Hauterkrankungen, die das klinische Erscheinungsbild von Tinea pedis hervorrufen können, in Bekleidungsstücken.

11. Verfahren zur Herstellung der Zusammensetzung nach einem der Ansprüche 1 bis 7, dadurch gekennzeichnet, daß die Bestandteile auf geeignete Weise gemischt werden.

## Claims

1. Composition characterised in that it contains constituent parts of the foliage of plants of the genus equisetum and of the foliage and/or the blossom of plants of the genus lavandula.

2. Composition according to claim 1 characterised in that the constituent parts of plants of the genus equisetum have been obtained by extraction and that the constituents of the genus lavendula are a flower oil.

3. Composition according to claim 1 or claim 2 characterised in that the extract was obtained with 30% (vol/vol) aqueous alcohol.

4. Composition according to claims 1 to 3 characterised in that the plants of the genus equisetum are plants of the types equisetum arvense and/or equisetum hismale and the plants of the genus lavandula are plants of the type lavandula officinelis chaix et vill (= lavandula angustifolia mill; real lavender), lavandula spica (= lavandula latifolia vill.) and/or lavandula hybrida rev.

5. Composition according to one of the claims 1 to 4 characterised in that the ratio of equisetum constituent parts to the lavandula constituent parts is in the range of 15:100 to 100:15 parts (weight/weight).

6. Composition according to claims 1 to 5 characterised in that they contain at least a carrying agent.

7. Composition according to claim 6 characterised in that the carrying agent is one which is acceptable for an external pharmaceutical and/or cosmetic application.

8. Application of the composition according to one of the claims 1 to 7 in the manufacture of a medication as a prophylactic against and to combat dermatomykoses, especially dermatophytoses, skin ailments having the clinical appearance of tinea pedis and skin ailments at other points on the body produced by their agents.

9. Application of the composition according to one of the claims 1 to 7 in the manufacture of a medication or cosmetic for combating bromohidrosis, sweating and in the manufacture of a deodorant.

10. Application of the composition according to one of the claims 1 to 7 in the manufacture of a medium to combat dermatomykosis agents and agents causing skin ailments which can produce the clinical appearance of tinea pedis, in items of clothing.

11. Process for the manufacture of the composition according to one of the claims 1 to 7 characterised in that the constituents parts are mixed in a suitable manner.

## Revendications

1. Composition, caractérisée en ce quelle comprend des composants de l'herbe de plantes du genre Equisetum et de l'herbe et/ou des fleurs de plantes du genre Lavandula.

2. Composition selon la Revendication 1, caractérisée en ce que les composants de plantes du genre Equisetum sont obtenus par extraction et en ce que les composants du genre Lavandula sont une essence de fleurs.

3. Composition selon la Revendication 1 ou 2, caractérisée en ce que l'extrait est obtenu par extraction avec un alcool aqueux à 30 % (vol/vol).

4. Composition selon l'une des Revendications 1 à 3, caractérisée en ce que les plantes du genre Equisetum sont des plantes du type Equisetum arvense et/ou Equisetum hiemale, et en ce que les plantes du genre Lavandula sont des plantes du type Lavandula officinalis Chaix et Vill. (= Lavandula angustifolia Mill. ; lavande véritable), Lavandula spica (= Lavandula latifolia Vill.) et/ou Lavandula hybrida Rev.

5. Composition selon l'une des Revendications 1 à 4, caractérisée en ce que le rapport entre les composants d'Equisetum et les composants de Lavandula est compris dans la gamme allant de 15:100 à 100:15 parties en masse.

6. Composition selon l'une des Revendications 1 à 5, caractérisée en ce qu'elle comprend au moins un véhicule.

7. Composition selon la Revendication 6, caractérisée en ce que le véhicule est un véhicule acceptable du point de vue pharmaceutique et/ou cosmétique pour une application externe.

8. Utilisation de la composition selon l'une des Revendications 1 à 7, pour la fabrication d'un médicament destiné à prévenir, par application externe, et à lutter contre les dermatomycoses, en particulier les dermatophytoses, les maladies cutanées présentant les manifestations cliniques de Tinea pedis, et les maladies cutanées provoquées par leurs agents causals sur d'autres parties du corps.

9. Utilisation de la composition selon l'une des Revendications 1 à 7, pour la fabrication d'un médicament ou d'un produit cosmétique destiné à lutter contre la bromhydrose, contre la sudation, et pour la fabrication d'un déodorant.

10. Utilisation de la composition selon l'une des Revendications 1 à 7, pour la fabrication d'un moyen destiné à lutter contre les agents causals de dermatomycoses et les agents causals de maladies cutanées, qui peuvent provoquer les manifestations cliniques de Tinea pedis, dans des vêtements.

11. Procédé de fabrication de la composition selon l'une des Revendications 1 à 7, caractérisé en ce que les composants sont mélangés de façon appropriée.
